# EUROPEAN PATENT SPECIFICATION

(11) **EP 1 227 857 B1**
(45) Date of publication and mention of the grant of the patent: **02.03.2005**
(21) Application number: 00978454.7
(22) Date of filing: 06.11.2000
(51) Int. Cl.: A61M 25/00

(54) **AORTIC CANNULA WITH SPOON-SHAPED LIP**
AORTENKANÜLE MIT EINER LÖFFELFÖRMIGEN ENDLIPPE
CANULE AORTIQUE POURVUE D'UNE LEVRE EN FORME DE CUILLERE

(30) Priority: 09.11.1999 US 436160; 20.12.1999 US 467326
(43) Date of publication of application: 07.08.2002
(73) Proprietor: Grooters, Ronald K., West Des Moines, IA 50266-6627 (US)
(72) Inventor: Grooters, Ronald K., West Des Moines, IA 50266-6627 (US)
(74) Representative: Parry, Christopher Stephen
(86) International application number: PCT/US2000/030788
(87) International publication number: WO 2001/034239

(56) References cited:
- EP-A- 0 653 191
- DE-A- 19 645 183
- US-A- 5 084 033
- US-A- 5 290 267
- US-A- 5 876 383

## Description

The present invention relates generally to medical devices and, in particular, aortic cannulas. Aortic cannulas are used to return blood to the aorta while the heart is by-passed during heart surgery. These cannulas are purposely made with small diameters to minimize disruption to the aorta, which in many heart surgery patients have advanced complex atherosclerotic plaque with adherent blood from bithrombi. Some conventional cannulas have a single discharge opening for the blood. It is desirable to eliminate or minimize impact onto the aortic wall of the blood exiting the cannula.

Aortic cannulas generally comprise an elongated tube having a terminal end. In at least some styles of conventional cannulas, a single opening is provided in the terminal end which provides a single stream of blood exiting the cannula and entering the aortic arch. Due to the small diameter of the cannula, the flow velocity of the blood through the single opening in the terminal end of the cannula is extremely high resulting in "jet" flow. The fluid pressure at the discharge end of the prior art cannula is also high. It is believed that the force of this jet stream of blood dislodges atherosclerotic plaque and/or adherent thrombi from the walls of the aorta, causing embolisms and strokes.

Attempts in the art to prevent embolisms resulting from cannulation have included designing the cannula in order to reduce the velocity of blood exiting the terminal end. For instance, U.S. Pat. No. 5,354,288 describes a cannula having a conical diffuser placed toward the proximal end of the cannula. The cannula includes several outlet openings in the sidewall to permit blood deflected by the diffuser to flow out of the cannula. This cannula design, however, still directs blood toward the sides of the aortic arch wherein the atherosclerotic plaque usually lies. Thus, the patient is still susceptible to embolisms and strokes.

US-A-5,876,383 discloses an aortic cannula comprising an elongate tube having a longitudinal axis and a terminal end with an opening therein and having a spoon-shaped lip extending from the terminal and adjacent the opening beyond the perimeter of the tube.

A primary objective of the present invention is the provision of an improved aortic cannula which disperses blood into the aorta with little, if any, contact on the aortic wall.

The invention provides an aortic cannula comprising an elongated tube having a longitudinal axis and a terminal end with an opening therein and having a spoon-shaped lip extending from the terminal end adjacent the opening beyond the perimeter of the tube; characterised in that the part of the lip extending beyond said perimeter is concave in all directions so as to discharge blood from the cannula in a hollowed stream.

An embodiment of the present invention provides an improved aortic cannula which has an internal bead adjacent the terminal end to facilitate blood dispersion from the cannula.

Preferably, the cannula discharges the blood in a U-shaped stream.

Another embodiment of the present invention provides a cannula which discharges the blood in a direction centrally aligned with the aortic lumen so as to avoid or minimize impact on the aortic wall.

A further embodiment of the present invention provides an improved aortic cannula having a discharge opening with an upper end having an inverted V-shaped contour to facilitate dispersion of blood from the cannula.

Embodiments of the invention are described below with reference to the accompanying drawings, in which:
FIG. **1** is a perspective view of the exemplary improved aortic cannula of the present invention.
FIG. **2** is a sectional view of the terminal end of the aortic cannula shown along lines 2-2 of FIG. **1**.
FIG. **3** is an elevation view of the exemplary embodiment of the aortic cannula taken along lines 3--3 of FIG. **2**.
FIG. **4** is a sectional view taken along lines 4-4 of Figure 1 showing an exemplary shape of the bead in the cannula of the present invention.
FIG. **5** is a schematic diagram of the heart and its primary blood vessels with the aortic cannula of the present invention inserted into the ascending aorta.
FIG. **6** is a schematic sectional view showing the initial and complete insertion positions of the cannula in the aorta.
FIG. **7** is a schematic view showing the cross sectional shape of the blood immediately after discharge from the cannula of the present invention.

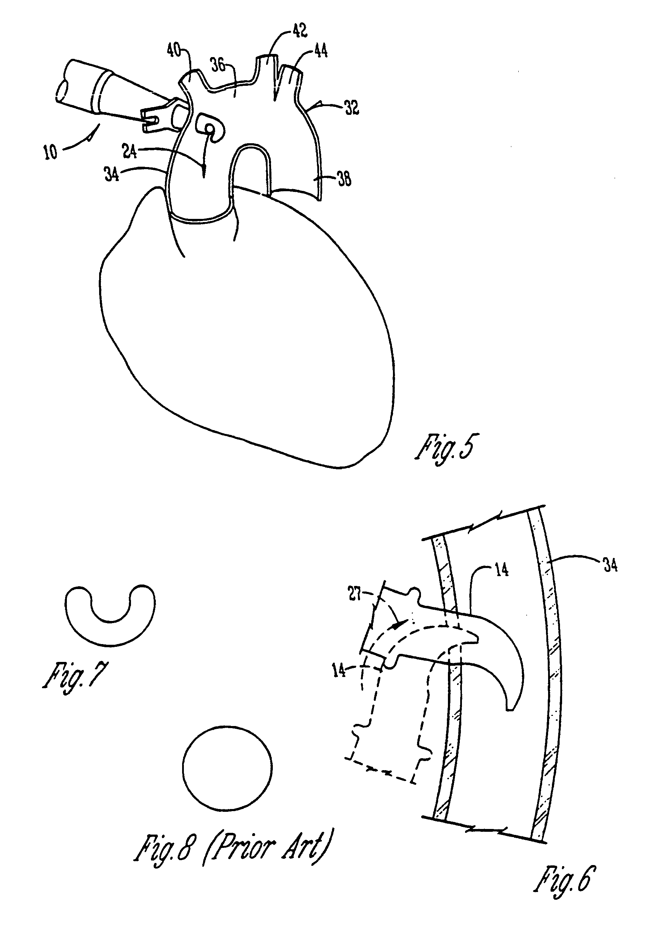

FIG. **8** is a view similar to FIG. **7** showing the jet stream of blood exiting the single opening of a prior art cannula.

The present invention is defined in claim 1 and will be described as it applies to its preferred embodiment.

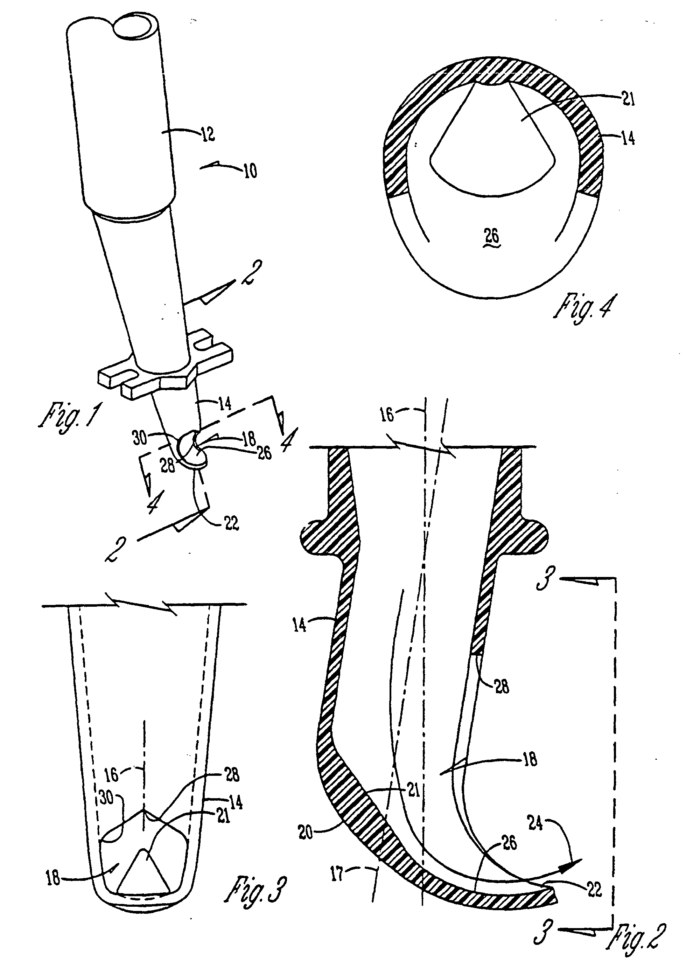

An exemplary embodiment of the improved aortic cannula of the present invention is generally designated by the reference numeral 10 in the drawings. With reference to Figures 1 and 2, the cannula 10 comprises an elongated tube 12 with a terminal end 14. As best seen in Figure 2, it is preferred that the terminal end 14 is angled or tilted slightly with respect to the longitudinal axis 16 of the tube 12. Preferably, by way of example and not limitation, the relative angle between the axis 17 of the terminal end 14 and the longitudinal axis 16 of the tube 12 is approximately 5° to 20°. The diameter of the tube 12 may taper toward the terminal end 14.

In one aspect of the present invention, an enlarged opening 18 is provided in the terminal end 14. The opening has a lower end 26 and an upper end 30. The upper edge of the opening 18 is curved, preferably with an inverted V-shape apex 28, as best seen in Figure 3.

The improved cannula has a spoon-shaped lip 22. A curved or ramped surface 20 directs blood along the spoon-shaped lip 22 extending from the terminal end 14. Preferably, the lip 22 extends from the terminal end 14 at approximately 70°-90° relative to the longitudinal axis 16 of the tube 12. The lip 22 extends beyond the perimeter of the tube 12, as best seen in Figure 2. The spoon-shaped lip 22 includes a side-to-side curvature as well as a front-to-back curvature. The spoon-shaped lip 22 causes the blood to exit the opening 18 in a hollowed stream, such as the U-shaped dispersion shown in Figure 7, without a wide band broadcast or fan. Thus, the exiting blood does not impact upon the wall of the ascending aorta 34, as shown in Figure 5, or the aortic arch 36, if used in that direction.

In a further aspect of the present invention, a bead 21 is formed on the ramped surface 20. Preferably, the bead 21 is wedge-shaped and integrally formed with the tube 12. An explanary embodiment is shown in figure 4, but other shapes and constructions will be apparent to one skilled in the art, such as a non-integral construction of the bead 21 in the tube 12.

In Figure 5, the aorta is designated by the reference numeral 32. The aorta 32 includes three main sections, the ascending aorta 34, the transverse aortic arch 36, and the descending aorta 38. The aortic arch 36 is the primary area where atherosclerotic plaque is found in patients needing heart bypass surgery. Branching from the aorta 32 are three large arteries, the innominate artery 40, the left carotid 42, and the left subclavian 44.

The opening 18, ramped surface 20, bead 21 and lip 22 allow the blood to be forced through the cannula 10 at a lower pressure. The large opening also reduces the velocity of the exiting blood. The ramped surface 20 and the lip 22 direct the blood toward the ascending aorta 34, as indicated by arrow 24, at an angle substantially 70°-90° from the longitudinal axis 16 of the tube 12. The blood may alternately be directed toward the aortic arch 36. Without the extended lip 22, which in effect extends the lower edge 26 of the opening 18 beyond the top edge 28 of the opening, the ramped surface 20 alone will only direct the exiting blood at an angle approximately 45° from the longitudinal axis 16 of the tube 12. The blood exits the opening 18 in a narrow hollow stream axially directed in the lumen of the ascending aorta 34 or aortic arch 36, thereby avoiding impact with the wall of the aorta. The bead 21 facilitates dispersion of the blood by interrupting the linear flow path through the tube 12. The inverted V-shaped apex 28 of the opening 18 also permits blood to exit the opening 18 adjacent the upper edge 30 thereof, and mix with the blood passing along the lip 22, thereby facilitating dispersion. The large size of the opening 18 also decreases the velocity of the blood exiting from the cannula 10.

After an incision is made by the surgeon in the ascending aorta 34, the cannula 10 is positioned such that the tube 12 is relatively close to the aorta. The end of the lip 22 is inserted into the incision and the cannula 10 is rotated upwardly as indicated by the arrow 27 in Figure 6, such that the tube 12 extends away from the aorta 32. The lip spreads and opens the incision for quick and easy insertion of the cannula terminal end 14, as seen in Figure 5. In Figure 6, the initial insertion position of the lip 22 is shown in broken lines, while the final insertion position of the terminal end 14 is shown in solid lines. This process is reversed for removal of the cannula 10 from the aorta 32.

Thus, the aortic cannula 10 of the present invention is quickly and easily inserted through a minimally sized incision in the aorta 32, thereby reducing risk of damage to the aortic wall and optimizing patient recovery. Potential damage to the wall of the aorta is also reduced since the spoon-shaped lip 22 eliminates or minimizes impact of exiting blood onto the aorta wall. Furthermore, by directing blood flow centrally along the aortic lumen, the improved cannula 10 reduces the chance that the plaque will become dislodged during cardiac bypass surgery, and thus, helps to reduce the risk of embolism and strokes. In prior art cannulas, the blood is directed towards the aortic wall and thus may dislodge plaque, which then can enter the blood stream and cause a stroke.

Whereas the invention has been shown and described in connection with the preferred embodiments thereof, it will be understood that many modifications, substitutions, and additions may be made which are within the scope of the following claims. From the foregoing, it can be seen that the present invention accomplishes at least all of the stated objectives.

## Claims

1. An aortic cannula (10) comprising an elongated tube (12) having a longitudinal axis (16) and a terminal end (14) with an opening (18) therein and having a spoon-shaped lip (22) extending from the terminal end (14) adjacent the opening (18) beyond the perimeter of the tube; **characterised in that** the part of the lip extending beyond said perimeter is concave in all directions so as to discharge blood from the cannula (10) in a hollowed stream.

2. The cannula of claim 1, wherein the lip (22) extends approximately 70°-90° relative to the longitudinal axis (16).

3. The cannula of any preceding claim, wherein the opening (18) is disposed on the tube to direct blood toward the ascending aorta.

4. The cannula of any preceding claim, wherein the opening (18) has an upper end (30) with an inverted V-shaped contour.

5. The cannula of any preceding claim, wherein the tube (12) has a raised bead (21) adjacent the terminal end (14) to facilitate dispersion of blood from the opening (18).

6. The cannula of any preceding claim, wherein the terminal end (14) of the tube (12) is angled with respect to the longitudinal axis (16).

## Patentansprüche

1. Aortenkanüle (10) mit einem länglichen Rohr (12), das eine Längsachse (16) und ein Abschlussende (14) mit einer darin ausgebildeten Öffnung (18) aufweist, und mit einer löffelförmigen Lippe (22), die sich von dem Abschlussende (14) im Bereich der Öffnung (18) über den Umfang des Rohres hinaus erstreckt, **dadurch gekennzeichnet, dass** der sich über den Umfang hinaus erstreckende Teil der Lippe in allen Richtungen konkav ist, so dass Blut aus der Kanüle (10) in einem eine Einsenkung aufweisenden Strahl austritt.

2. Kanüle nach Anspruch 1, bei der sich die Lippe (22) mit etwa 70° - 90° relativ zu der Längsachse (16) erstreckt.

3. Kanüle nach einem der vorangehenden Ansprüche, bei der die Öffnung (18) an der Röhre ausgebildet ist, um Blut in Richtung der aufsteigenden Aorta zu lenken.

4. Kanüle nach einem der vorangehenden Ansprüche, bei der die Öffnung (18) ein oberes Ende (30) mit einer umgekehrten V-förmigen Gestalt hat.

5. Kanüle einem der vorangehenden Ansprüche, bei der die Röhre (12) im Bereich des Abschlussendes (14) eine erhabene Verdickung (21) aufweist, um die Abgabe von Blut aus der Öffnung (18) zu erleichtern.

6. Kanüle nach einem der vorangehenden Ansprüche, bei der das Abschlussende (14) des Rohres (12) in Bezug auf die Längsachse (16) winklig angeordnet ist.

## Revendications

1. Canule aortique (10) comportant un tube allongé (12) ayant un axe longitudinal (16) et une extrémité terminale (14) avec une ouverture (18) dans celle-ci et ayant une lèvre en forme de cuillère (22) s'étendant à partir de l'extrémité distale (14) à proximité de l'ouverture (18) au-delà du périmètre du tube, **caractérisée en ce que** la partie de la lèvre s'étendant au-delà dudit périmètre est concave dans toutes les directions de manière à évacuer le sang de la canule (10) en un flux creusé.

2. Canule selon la revendication 1, dans laquelle la lèvre (22) s'étend à approximativement 70° à 90° par rapport à l'axe longitudinal (16).

3. Canule selon l'une quelconque des revendications précédentes, dans laquelle l'ouverture (18) est disposée sur le tube pour diriger le sang vers l'aorte montante.

4. Canule selon l'une quelconque des revendications précédentes, dans laquelle l'ouverture (18) a une extrémité supérieure (30) ayant un contour en forme de V inversé.

5. Canule selon l'une quelconque des revendications précédentes, dans laquelle le tube (12) a un bourrelet en relief (21) à proximité de l'extrémité terminale (14) pour faciliter la dispersion du sang à partir de l'ouverture (18).

6. Canule selon l'une quelconque des revendications précédentes, dans laquelle l'extrémité terminale (14) du tube (12) est inclinée par rapport à l'axe longitudinal (16).
